# EUROPEAN PATENT SPECIFICATION

(11) **EP 0 853 878 B1**
(45) Date of publication and mention of the grant of the patent: **19.09.2001**
(21) Application number: 98830013.3
(22) Date of filing: 16.01.1998
(51) Int. Cl.: A01K 67/033, C05F 17/02, C05F 17/00

(54) **Earthworm breeding**
Das Züchten von Regenwürmern
L'élevage de lombrics

(30) Priority: 16.01.1997 IT BS970004
(43) Date of publication of application: 22.07.1998
(73) Proprietor: Ecoflor S.r.l., 25123 Brescia (IT)
(72) Inventor: Facchi, Ferdinando, 25128 Brescia (IT)
(74) Representative: Manzoni, Alessandro

(56) References cited:
- WO-A-93/10060
- DE-A- 3 331 381
- FR-A- 2 297 556
- FR-A- 2 733 170
- PATENT ABSTRACTS OF JAPAN vol. 95, no. 6, 31 July 1995 & JP 07 069765 A (HASAKA MASARU), 14 March 1995,

## Description

The present invention refers to the earthworm breeding sector and in particular to a vermicomposting frame plant.

As is well known, earthworm breeding or also vermicomposting consists in a transformation of organic liquid, muddy or solid waste by earthworms into humus to be used as a compost.

The waste to be treated can be mud from civil, farm and food cleaners, breeding farm waste, green plant trimmings, industrial waste from textile, dyeing and paper plants etc. besides urban waste.

According to the most widespread techniques, earthworm breeding can be carried out in silos or in containers having a perforated inner base as disclosed, for example, in WO-A-93/10060. But, most frequently earthworm breeding is conducted in horizontal frames placed on the earth outside or indoors. These frames may be enclosed using small prefabricated walls or lateral enclosures, earth banks or the like and usually separated by service lanes. However they have the problem of eliminating water from the atmosphere and liquids which form during breeding.

The earth under the layer of bedding is not always capable of absorbing this liquid and often the fict that this liquid is able to penetrate regularly into the earth is not acceptable. Furthermore, if the liquid stagnate in the bedding it may limit or compromise the efficacy of the vermicomposting process.

The document DE-A-3331381 discloses a method of hydroculture using single transportable troughs, which are loaded of culture loam and which are provided with pipes for both drainage and imgation. This document, however, does not discloses nor suggest any technique of earthworm breeding, even if the troughs are provided with drainage pipes.

The aim of this invention is to remedy these drawbacks and to create the best conditions in vermicomposting breeding plant which ensure collection and drainage of the liquids from the bottom of the bedding layer, despite the earth below being permeable or not.

According to this invention, this aim is reached with the use for a vermicomposting breeding operation of a plant in accordance with the claim 1, and accordingly:
. by waterproofing the bottom and the sides of each frame to avoid infiltration of liquids into the earth below and by sloping the bottom at one end least;
. by laying a micro-slotted drainage pipe in the lowest side of the bottom and along the whole length of the frame;
. by covering the drainage pipe with gravel; and
. by connecting the drainage tube to an end manifold which drains into a collection tank.

Further details of the invention will become more obvious from the rest of the description formulated in reference to the drawings enclosed, in which:
Fig. 1 shows a schematic top view of a vermicomposting breeding plant with several frames;
Fig. 2 shows a cross-section of a frame along the II-II line in Fig. 1; and
Fig. 3 shows a blown-up detail of Fig. 2.
In these drawings, 10 indicates the various frames of a vermicomposting breeding plant and arranged to receive the bedding mentioned above to be transformed into humus. The frames 10 are enclosed on each side by low walls, prefabricated elements 11, enclosures, embankments or some such other means and separated by service lanes 12.
Along each frame 10 bases 13 may be provided for poles 14 to support a possible greenhouse type roofing 14'

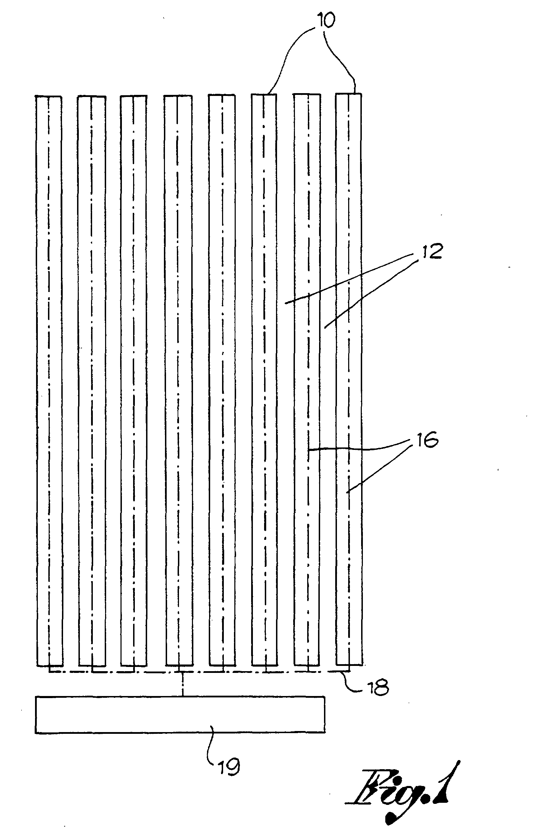

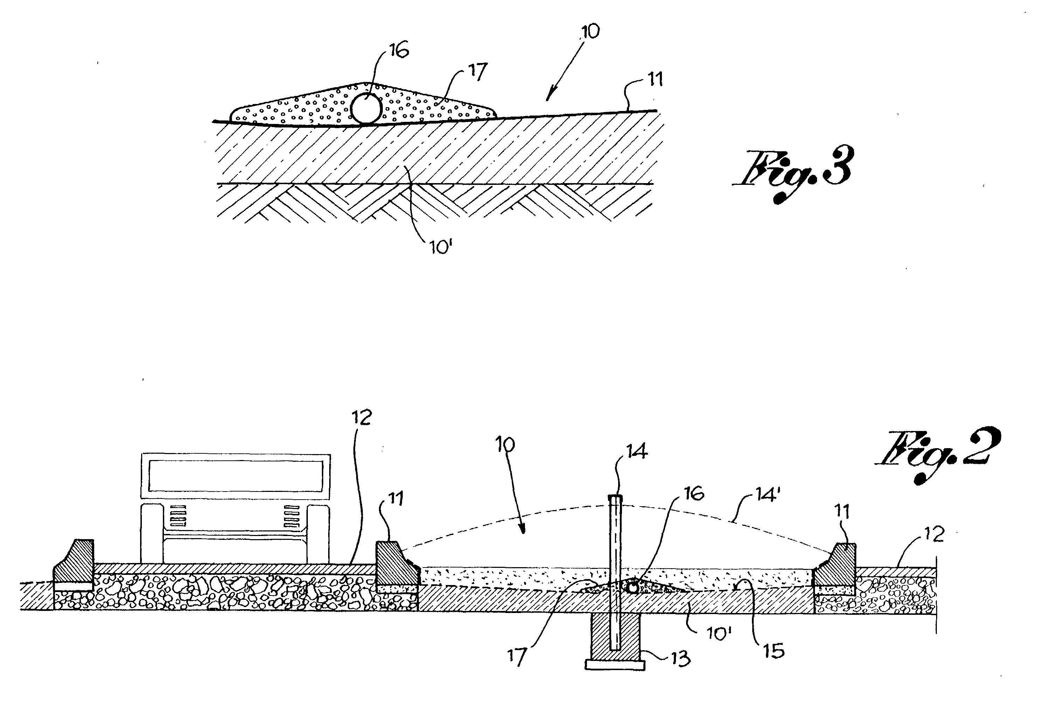

The bottom 10' of each frame is sloping to one side or, as shown in the drawing, towards the center and is waterproofed. Waterproofing may be achieved by placing a layer of pressed clay or some other waterproofing material on the bottom, by cementing the bottom or as shown in the diagram using a waterproof sheet 15 which is laid so as to cover the sides of the frame as well.

When used, the waterproofed sheet 15, can be made of polyethylene some other plastic material or suitable non woven fabric acting as a base for collecting the water which sifts through the bedding layer.

In the lowest part of the sloping area on the bottom of the waterproofed bottom 10' there is a micro-slotted drainage pipe 16 which travels longitudinally along the whole length of each singular frame. The pipe 16 is covered by a layer of gravel 17 and is used to collect and evacuate the water which accumulates and runs off the sloping waterproofed sheet.

Therefore, the water percolating through the bedding of each frame and the layer of gravel covering the drainage pipe is collected and evacuated by the pipe itself and piped to the tank or catch basin. All this without the water coming into contact and sifting into the earth underneath.

## Claims

1. The use for a vermicomposting breeding operation of a plant consisting of several frames (10), enclosed laterally either by small walls, prefabricated elements (11), enclosures, embankments or the like and arranged to receive the bedding to be transformed into humus, wherein each frame has bottom and sides waterproofed to avoid infiltration of liquid into the ground underneath and the bottom of the frame either slopes to one sides or towards the center, wherein in the lowest part of said waterproofed bottom at least one micro slotted drainage pipe (16) is laid which travels longitudinally along the whole length of each singular frame; and wherein each drainage pipe (16) is covered by a layer (17) of gravel.

2. The use for a vermicomposting breeding operation of a plant according to claim 1, where the drainage pipe (16) of the frames are connected to an end manifold to evacuate the drainage water into a tank or catch basin.

3. The use for a vermicomposting breeding operation of a plant according to claims 1 and 2, where the bottom (10') of each frame is waterproofed by creating a layer of pressed clay or other insulating material or a cement surface with the drainage pipe (16) laying on this layer or surface.

4. The use for a vermicomposting breeding operation of a plant according to claims 1 and 2, where the bottom and sides of each frame is covered by a waterproofed sheet (15) and the drainage tube (16) is placed an this sheet.

## Patentansprüche

1. Für eine Regenwurmzucht die Nutzung einer Anlage mit zahlreichen Streulagem (10), die seitlich durch Mäuerchen, Fertigteile (11), Einzäunungen, Aufschüttungen o.ä. begrenzt sind und dazu dienen, das in Humus umzuwandelnde Material aufzunehmen, wobei jedes Streulager einen abgedichteten Boden und abgedichtete Seitenpartien hat, um das Einsickern von Wasser in das darunterliegende Erdreich zu verhindern, und der Boden des Streulagers nach einer Seite oder zur Mitte hin abfallend ist und an der niedrigsten Stelle des besagten abgedichteten Bodens mindestens ein mit Mikro-Ritzen versehenes Entwässerungsrohr (16) gelegt ist, das sich in Längsrichtung über die gesamte Länge jedes einzelnen Streulagers erstreckt; dabei ist jedes Entwässerungsrohr (16) durch eine Schicht (17) Kies bedeckt.

2. Für eine Regenwurmzucht die Nutzung einer Anlage gemäß Anspruch 1, bei der die Entwässerungsrohre (16) der Streulager an einen Endsammelkanal angeschlossen sind, der zum Ablass des Dränungswassers in ein Auffangbecken oder einen Sammelbehalter dient.

3. Für eine Regenwurmzucht die Nutzung einer Anlage gemäß den Patentansprüchen 1 und 2, bei der der Boden (10') jedes einzelnen Streulagers abgedichtet ist durch den Einsatz einer Schicht aus gepresstem Lehm oder aus einem anderen Sperrstoff oder durch den Einsatz einer zementierten Oberfläche, wobei das Entwässerungsrohr (16) auf der besagten Schicht oder Oberfläche aufliegt.

4. Für eine Regenwurmzucht die Nutzung einer Anlage gemäß den Patentansprüchen 1 und 2, bei der der Boden und die Seitenpartien jedes einzelnen Streulagers mit einer Feuchtigkeitsdämmschicht (15) bedeckt sind und das Entwässerungsrohr (16) auf dieser Feuchtigkeitsdämmschicht angebracht ist.

## Revendications

1. Utilisation dans une activité de lombricolture d'une station comprenant de nombreuses litières (10), délimitées latéralement par des murets, des éléments préfabriqués (11), des clôtures, des terre-pleins ou similaires, et destinées à recevoir la matière à transformer en humus, chaque litière ayant un fond et des côté imperméabilisés pour éviter les infiltrations de liquide dans le terrain sous-jacent et le fond de la litière est en pente d'un côté ou au centre, où dans la partie plus basse dudit fond imperméabilisé est placé au moins un tube microfissuré de drainage (16) qui s'étend longitudinalement sur toute la longueur de chaque litière, et où chaque tube de drainage (16) est couvert d'une couche (17) de gravier.

2. Utilisation dans une activité de lombricolture d'une station conforme à la revendication 1, où les tubes de drainage (16) des litières sont reliés à un collecteur terminal d'évacuation de l'eau de drainage vers une cuve ou un réservoir de récolte.

3. Utilisation dans une activité de lombricolture d'une station conforme aux revendications 1 et 2, où le fond (10') de chaque litière est imperméabilisé moyennant la création d'une couche d'argile pressée ou autre matériau isolant ou d'une surface cimentée avec le tube de drainage (16) qui s'appuie sur ladite couche ou surface.

4. Utilisation dans une activité de lombricolture d'une station conforme aux revendications 1 et 2, où le fond et les côtés de chaque litière sont recouverts d'une toile de revêtement imperméabilisée (15) et le tube de drainage (16) est placé sur cette toile de revêtement
